# EUROPEAN PATENT APPLICATION

(11) **EP 2 055 283 A1**
(43) Date of publication of application: **06.05.2009**
(21) Application number: 07021203.0
(22) Date of filing: 30.10.2007
(51) Int. Cl.: A61F 13/62

(54) **Fastening tab**

(71) Applicant: 3M Innovative Properties Company, St. Paul MN 55133-3427 (US)
(72) Inventor: Petersen, Johann, 41516 Grevenbroich (DE); Oertel, Ralf, 41462 Neuss (DE)
(74) Representative: Vossius & Partner

(57) **Abstract**

The invention relates to improved fastening tabs for disposable products such as diapers, to disposable products comprising said fastening tabs, and to methods of manufacturing said disposable products. According to one embodiment, the fastening tab for comprises a backing having a manufacturer's end and an opposite user's end. The fastening tab is adapted to be secured to the disposable product at its manufacturer's end, and the user's end comprises a fingerlift. The tab further comprises a mechanical fastening element attached to the user's end of the backing. The user's end is folded in a Z-shape such that the mechanical fastening element is at least partially covered by a portion of the user's end. The fastening tab is adapted so that, upon application of a pulling force onto the user's end of the fastening tab, the Z-shape is released and the covering of the mechanical fastening element is removed so that the mechanical fastening element is exposed.

## Description

### Field of the Invention

The invention relates to fastening tabs for disposable products, to disposable products comprising said fastening tabs and to methods of manufacturing said disposable products.

### Background of the Invention

Nowadays, disposable products, such as diapers, are widely used not only in the sector of child care, but also for adults suffering from incontinence. These disposable products are typically reclosable by fastening tabs which are easy to handle and which provide a reliable fixation.

Generally, such fastening tabs either comprise an adhesive-based closure mechanism or a mechanical closure mechanism or a combination of both. Fastening tabs relying on a mechanical closure mechanism typically comprise a mechanical fastening element with hooks, said tab being attached to a rear waist portion of the diaper or to a diaper ear. If the diaper is being used the mechanical fastening element of the fastening tab can be attached to a landing zone on a front waist portion of the diaper. For this purposes the landing zone comprises a surface with a loop material being adapted to engage with the hooks of the fastening element. Alternatively, the loop material may, e.g., be an exposed non-woven material suitable to engage with the hooks of the fastening element. In that case a landing zone as a separate element may be omitted.

Prior to the use of the diaper, the fastening tab is generally secured to the inside of the rear waist portion (i.e., the topsheet) or to the inside of the diaper ear so that the fastening tab does not protrude from the diaper chassis and interfere with the machinery used in the manufacturing process of the diaper. This is achieved, e.g., by providing a non-woven on the inside to which the fastening tab can attach by engagement of the fastening element with the fibers of the non-woven. For example, EP 0 974 326 A1 and EP 0 941 728 A2 disclose such diaper closure systems.

However, with this solution, under certain combinations of weak fiber bonds of the nonwoven material and a hook material with strong/good engagement properties, some of the fibers may get stuck within the hook structure of the fastening element upon disengagement of the mechanical fastening element. Not only may this result in an unaesthetic appearance of the diaper ear, but it may also lead to inactivation of the hook structure due to coverage with fibres.

### Summary of the Invention

It is therefore an object of the present invention to provide an improved fastening tab avoiding the above-mentioned disadvantages. This object is achieved by the features of the claims.

According to the present invention, a fastening tab for a disposable product comprises a backing having a manufacturer's end and an opposite user's end, wherein the fastening tab is adapted to be secured to the disposable product at its manufacturer's end, and the user's end comprises a fingerlift; a mechanical fastening element attached to the user's end of the backing; and a fingerlift portion protruding from the user's end of the backing. The user's end is folded in a Z-shape such that the mechanical fastening element is at least partially covered by a portion of the user's end. Further, upon application of a pulling force onto the user's end of the fastening tab, in particular at the distal end of the fingerlift, i.e., in a direction in the plane of the fastening tab, the Z-shape is released and the covering of the mechanical fastening element is removed so that the mechanical fastening element is exposed.

The (male) mechanical fastening element preferably comprises a hook structure adapted to mechanically engage with a corresponding (female) loop structure on a landing zone of a disposable product.

In one embodiment, the fingerlift protrudes from the backing of the fastening tab. In another embodiment, the fingerlift may be provided as an extension of the backing distal of the mechanical fastening element. In the latter embodiment, the backing as such forms the fingerlift. To this end a blanking film may be attached to the typically adhesively covered surface bearing the mechanical fastening element.

It is preferred that the fingerlift only relatively loosely covers the mechanical fastening element so that the covering of the mechanical fastening element can be easily removed. In other words, the fingerlift is not strongly attached to mechanical fastening element, e.g., by using a well suited hook-and-loop system with components being particularly selected for good and reliable engagement. Rather, the mechanical fastening element and/or the covering fingerlift material is chosen such that the fingerlift is basically just covering the mechanical fastening element with minimal holding power. This may be achieved, for example, with an appropriate non-woven material at the fingerlift and/or part of the backing which will be engaged by the hook structure. The Z-shape may alternatively or additionally be retained by means of spot bonding and/or welding.

Advantageously, the pulling force onto the user's end sufficient for releasing the Z-shape and exposing the mechanical fastening element is between 0.5 N and 10 N.

The Z-folded user's end of the fastening tab generally comprises a base portion, a middle portion and a top portion. In one embodiment the base portion is formed by the backing including the mechanical fastening element, and the middle and top portions are formed by the fingerlift portion and/or the backing. In another embodiment the base portion is formed by the backing, the middle portion is formed by the backing including the mechanical fastening element, and the top portion is formed by the fingerlift portion and/or the backing.

As stated above, the fingerlift may also be an integral part of the backing. If, for instance, the backing comprises a layer of adhesive, the fingerlift may be formed by omitting the adhesive in a distal-most region at the user's end. Alternatively, the adhesive may be covered with a blanking film in order to provide a non-sticky fingerlift portion.

In some cases, the fastening tab may further comprise a release tape attached to the backing.

According to another embodiment of the present invention, a fastening tab for a disposable product comprises a backing having a manufacturer's end and an opposite user's end, wherein the fastening tab is adapted to be secured to the disposable product at its manufacturer's end.

The fastening tab further comprises a mechanical fastening element attached to the backing at its user's end, and a release tape having a first leg firmly attached to the backing and a second leg folded around a fold line over onto the mechanical fastening element, wherein the second leg is releasably attached to the mechanical fastening element to cover at least partially the mechanical fastening element.

Preferably, the second leg of the release tape comprises a non-woven material. It is also preferred that only a portion of the second leg is adapted to be attached to the disposable product. For example, the second leg of the release tape may comprise an adhesive in a first portion adjacent the fold line for attachment to the disposable product and a second portion free of adhesive covering the mechanical fastening element.

In another embodiment, the second leg may be entirely free of adhesive and attached to an additional adhesive layer, which is provided to the top sheet of a diaper.

The present invention also relates to a roll of fastening tape comprising a plurality of fastening tabs according to any of the above-mentioned embodiments, wherein the fastening tabs are arranged on the roll in a cross-direction so that individual fastening tabs can be cut from the fastening tape.

According to a further aspect of the present invention a diaper comprising a back sheet, a top sheet, and an absorbent core therebetween is provided. The diaper comprises a fastening tab according to any one of the above-mentioned embodiments, wherein the fastening tab is attached to the diaper at its manufacturer's end and the mechanical fastening element is at least partially covered so that the mechanical fastening element is disabled from fully engaging the top sheet of the diaper. Moreover, a landing zone with a co-operating fastening element, e.g., a loop structure, is provided at the diaper as well.

The diaper may further comprise a diaper ear, which may advantageously be elastic, wherein the fastening tab is attached to the diaper ear at a distal end portion thereof. The user's end of the fastening tab is preferably attached releasably to the diaper or diaper ear.

According to another embodiment of the present invention, a diaper comprises a back sheet, a top sheet, an absorbent core therebetween, and a fastening tab. The fastening tab comprises a backing having a manufacturer's end and an opposite user's end, wherein the fastening tab is secured to the diaper at its manufacturer's end. A mechanical fastening element is attached to the backing at its user's end. Further, a covering strip, e.g., made of a non-woven material, is provided for releasably covering the mechanical fastening element. The covering strip is adhered at least partially to the top sheet of the diaper; wherein the attachment between covering strip and top sheet is substantially stronger than the attachment between covering strip and mechanical fastening element.

Thus, if the user's end of the fastening tab is pulled by a user in order to close the diaper, the mechanical fastening element and covering strip disengage, the covering strip remains at the top sheet of the diaper and the mechanical fastening element is exposed for use.

It is preferred that only a portion of the covering strip is adhered to the back sheet of the diaper. Thus, the mechanical properties of the diaper chassis or ear are influenced by the covering strip to a small extent only.

The diaper may further comprise an elastic diaper ear, wherein the fastening tab is secured to the diaper ear and the covering strip is adhered to the inner surface of the diaper ear.

The present invention also relates to methods of manufacturing diapers. According to one embodiment, such a method comprises the steps of providing a diaper comprising a back sheet, a top sheet and an absorbent core therebetween; providing a fastening tab according to the abovementioned embodiments; attaching the fastening tab with its manufacturer's end to the diaper; and folding the user's end of the fastening tab around an edge of the diaper. Optionally, the method further comprises the step of releasably attaching the user's end of the fastening tab to the top sheet of the diaper.

According to another embodiment, the method comprises the steps of providing a diaper comprising a back sheet, a top sheet and an absorbent core therebetween; providing a fastening tab comprising a backing having a manufacturer's end and an opposite user's end, a mechanical fastening element attached to the backing at its user's end, and a covering strip for releasably covering the mechanical fastening element; securing the manufacturer's end of the fastening tab to the diaper; folding the user's end of the fastening tab around an edge of the diaper; and adhering said covering strip to the top sheet of the diaper; wherein the attachment between covering strip and top sheet is substantially stronger than the attachment between covering strip and mechanical fastening element.

These and other aspects of the present invention will be apparent from and elucidated with reference to the embodiments described hereinafter.

### Brief Description of the Drawings

- Fig. 1a: schematically shows an embodiment of a fastening tab according to the present invention;
- Fig. 1b: schematically shows the fastening tab of Fig. 1a being attached to and folded around a diaper ear according to the present invention;
- Fig. 1c: schematically shows the fastening tab of Fig. 1a attached to a diaper ear, being unfolded and ready for use;
- Fig. 2a: schematically shows another embodiment of a fastening tab according to the present invention;
- Fig. 2b: schematically shows the fastening tab of Fig. 2a being attached to and folded around a diaper ear according to the present invention;
- Fig. 2c: schematically shows the fastening tab of Fig. 2a attached to a diaper ear, being unfolded and ready for use;
- Fig. 3a: schematically shows another embodiment of a fastening tab for a diaper according to the present invention;
- Fig. 3b: schematically shows the fastening tab of Fig. 3a being attached to and folded around a diaper ear according to the present invention;
- Fig. 3c: schematically shows the fastening tab of Fig. 3a attached to a diaper ear, being unfolded and ready for use;
- Fig. 4a: schematically shows another embodiment of a fastening tab according to the present invention together with a diaper ear;
- Fig. 4b: schematically shows the fastening tab of Fig. 4a being attached to and folded around the diaper ear according to the present invention;
- Fig. 4c: schematically shows the fastening tab of Fig. 4a attached to the diaper ear being unfolded and ready for use;
- Fig. 5a: schematically shows two variations of the embodiment shown in Fig. 2a;
- Fig. 5b: schematically shows the fastening tab of Fig. 5a being attached to and folded around a diaper ear according to the present invention; and
- Fig. 5c: schematically shows the fastening tab of Fig. 5a attached to a diaper ear being unfolded and ready for use.

### Detailed Description of Preferred Embodiments

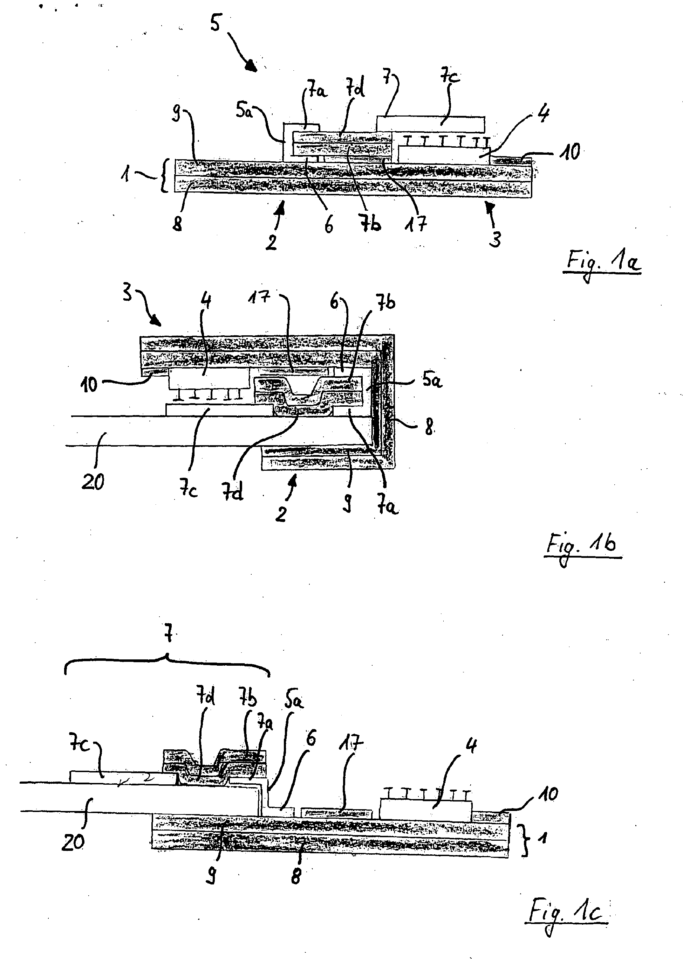

Fig. 1a schematically shows an embodiment of a fastening tab according to the present invention. The fastening tab comprises a backing 1 with a manufacturer's end 2 and a user's end 3. The backing 1 comprises a substrate layer 8 coated with a layer of adhesive 9. At the user's end 3, a mechanical fastening element 4 is attached to the backing 1. The fastening tab further comprises a release tape 5 having a first leg 6 firmly attached to the backing 1 and a second leg 7 folded around a fold line over onto the mechanical fastening element 4.

The release tape 5 may essentially comprise one piece, e.g., a film or non-woven material, that has an adhesive on it and is folded onto itself to provide the first and second legs. Preferably, the release tape 5 comprises several elements and/or portions. For instance, release tape 5 may comprise a center strip 5a comprising the first leg 6, a portion 7a of the second leg 7 and an inter-connecting portion therebetween. Attached to the second leg portion 7a is a strip 7b forming a bridge to a cover strip 7c. The bridging strip 7b is preferably covered by an adhesive layer 7d. This adhesive layer 7d attaches the bridging strip 7b at one end to the inside of the second leg portion 7a and at the opposite end to the cover strip 7c. A portion inbetween the attached ends of bridging strips 7b has the adhesive 7d exposed. The exposed adhesive 7d is used to irreleasably fix the second leg 7 to the diaper as shown in more detail in Figs. 1b and 1c. The center strip 5a and the bridging strip 7b are, for example, made of film materials. The cover strip 7c of the second leg 7 may comprise, e.g., a non-woven material which may be releasably attached to the mechanical fastening element 4. Alternatively, the cover strip 7c may also be formed from a film material.

An additional blanking film 17 may be provided on the adhesive 9 of backing 1, which blanking film 17 helps to avoid attachment of the second leg 7 to the backing 1. Alternatively, the substrate layer 8 may not be completely covered with the adhesive layer 9, but strip-coated instead. Thus, only (a) portion(s) of the substrate layer 8, where the adhesive is needed for attachment of further elements, is/are covered with the adhesive layer 9.

Fig. 1b schematically shows the fastening tab of Fig. 1a together with a diaper ear 20 of a diaper. The manufacturer's end 2 of the backing 1 is attached to the diaper ear 20 via adhesive layer 9. The user's end 3 of the fastening tab is folded around the diaper ear 20 and attached to it by the adhesive layer 7d of second leg 7 of release tape 5. Additionally or alternatively, the second leg portion 7a and/or the cover strip 7c may be covered with an adhesive layer for this purpose. Preferably, only a portion of the second leg 7, i.e., the portion of bridging strip 7b with exposed adhesive 7d, is adapted to be attached to the diaper ear.

In order to provide a fingerlift to a user, who intends to unfold the fastening tab for use, a portion of the adhesive layer 9 at the user's end 3 is covered with a blanking film 10. Thus, the user can easily grip the fastening tab, since this portion does neither stick to the diaper ear 20 nor to the user's finger.

The user may then release the user's end 3 from the diaper ear 20, thereby detaching the mechanical fastening element 4 from the second leg 7 of the release tape 5 and leaving the fastening tab in the situation shown schematically in Fig. 1c. In this position, the mechanical fastening element 4 is uncovered and ready for use, i.e., it may be attached to a landing zone comprising the respective female fastening element on a corresponding part of the diaper.

In the position shown in Fig. 1c it can also be appreciated that it is advantageous to attach only the portion of the second leg 7 with exposed adhesive 7d (and optionally second leg portion 7a) to the diaper ear 20, particularly in case of an elastic or stretchable diaper ear 20. Since in such a situation the second leg 7 of the release tape 5 is only partially attached to the diaper ear 20 it does not constrain the stretchability of the diaper ear any further than already at the attachment site of the manufacturer's end 2 of the backing 1. However, in some cases it may be necessary and/or advantageous that the portion of second leg 7, which is attached to the diaper ear 20, extends further than the portion of the backing 1 attached to the other side of the diaper ear 20.

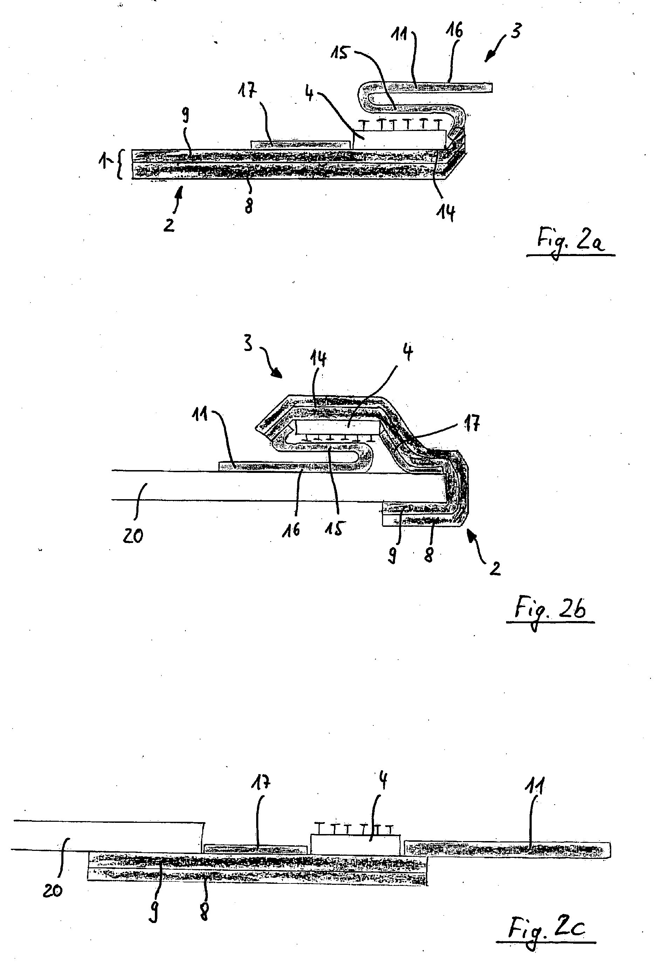

Fig. 2a schematically shows another embodiment of a fastening tab according to the present invention. Similar to the embodiment shown in Fig. 1a, the fastening tab comprises a backing 1 with a manufacturer's end 2 and a user's end 3. The backing 1 comprises a substrate layer 8 coated with a layer of adhesive 9. At the user's end 3, a mechanical fastening element 4 is attached to the backing 1 through adhesive layer 9. The fastening tab according to this embodiment further comprises an elongated fingerlift 11 protruding from the substrate layer 8 of the backing 1. The fingerlift 11 is folded into a general Z-shape, wherein the mechanical fastening element 4 is covered by the fingerlift 11. In particular, fingerlift 11 may be releasably attached to the mechanical fastening element 4. The folding may also be such as to cover the mechanical fastening element 4 only partly. The Z-shape may be retained by means of spot bonding or welding.

The Z-shape comprises a base portion 14 formed by the backing 1 including the mechanical fastening element 4, a middle portion 15 covering the mechanical fastening element 4 and a top portion 16. In this embodiment, the middle portion 15 and top portion 16 are formed by the fingerlift 11.

A blanking film 17 is preferably provided adjacent to the mechanical fastening element 4. The blanking film 17 is intended to avoid adherence of the fastening tab to the top sheet of the diaper in the storage configuration as shown in Fig. 2b. Alternatively, a strip-coated adhesive 9 may be used on the substrate layer 8.

According to Fig. 2b, the manufacturer's end 2 of the backing 1 is attached to the diaper ear 20 via the adhesive layer 9. The user's end 3 of the fastening tab is folded around the diaper ear 20 and attached to it, for example by an adhesive, by spot bonding or welding. Another option is that the mechanical fastening element 4 is only partially covered by the Z-fold. In that case, the mechanical fastening element 4 may partially engage with diaper ear 20. Upon application of a pulling force onto the user's end 3 of the fastening tab, the Z-shape can be released and the covering of the mechanical fastening element 4 is removed so that the mechanical fastening element 4 is exposed for use as shown in Fig. 2c.

Although not shown in Figs. 2a to 2c, the fastening tab may further comprise a release tape, similar to that described in relation to the embodiment according to Figs. 1a-1c, attached to the backing 1 as well as further adhesive layers, e.g., on a region of the fingerlift portion 11 in order to attach the latter to the diaper ear 20.

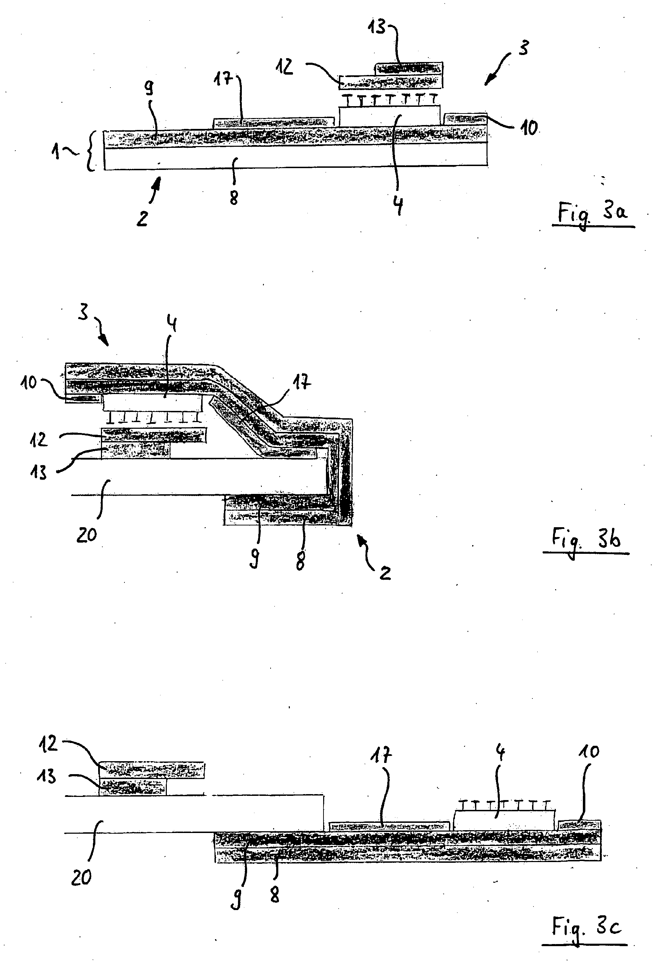

Fig. 3a schematically shows another embodiment of a fastening tab for a diaper according to the present invention. The fastening tab comprises a backing 1 with a manufacturer's end 2 and a user's end 3. The backing 1 comprises a substrate layer 8 coated with a layer of adhesive 9. At the user's end 3, a mechanical fastening element 4 and a blanking film 10 is attached to the backing 1. The fastening tab further comprises a covering strip 12, e.g., a non-woven or film material, and an adhesive layer 13 for releasably covering the mechanical fastening element 4. It is preferred that only a portion of the covering strip 12 is attached to the diaper ear 20. This may, for instance, be achieved by the adhesive layer 13 being smaller than the covering strip 12 as indicated in Figs. 3a to 3c. This is desirable in order to constrain the stretchability of the diaper ear 20 not more than necessary. The fastening tab preferably further comprises a blanking film 17. Alternatively, a strip-coated adhesive 9 may be used on the substrate layer 8.

As shown in Fig. 3b, the manufacturer's end 2 of the backing 1 is attached to the diaper ear 20 via adhesive layer 9. The user's end 3 of the fastening tab is folded around the diaper ear 20 and attached to it by the adhesive layer 13 of covering strip 12. According to this embodiment of the present invention, the attachment between covering strip 12 and diaper ear 20 is substantially stronger than the attachment between covering strip 12 and mechanical fastening element 4. Accordingly, if the user's end 3 of the fastening tab is being pulled off the diaper ear 20, the covering strip 12 remains attached to the diaper ear 20 and is disengaged from mechanical fastening element 4. This situation is shown in Fig. 3c.

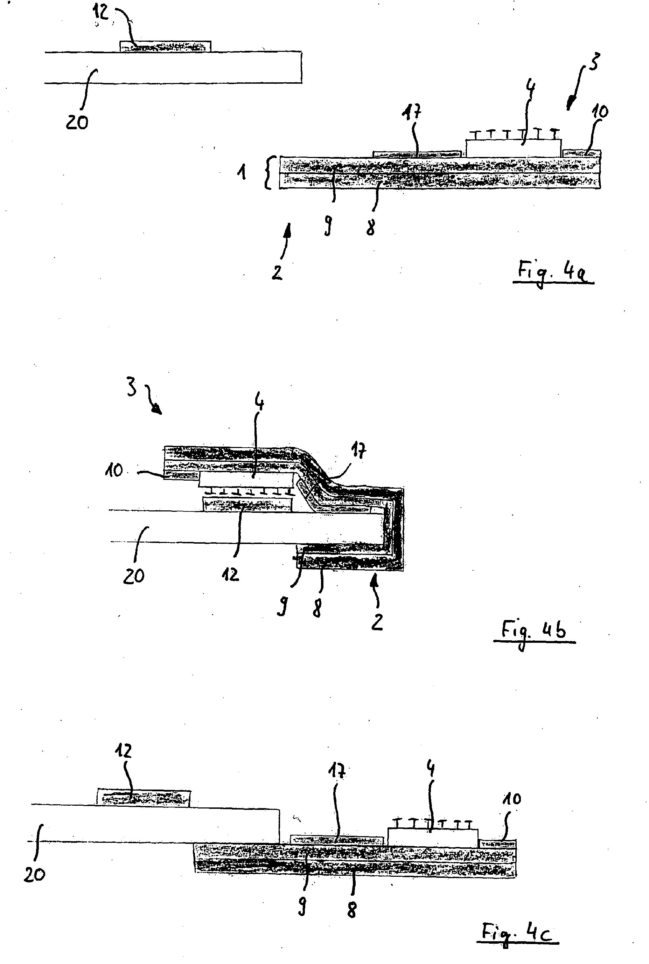

In Figs. 4a to 4c, another embodiment is illustrated which is similar to the one according to Figs. 3a to 3c. In this embodiment, the covering strip 12 is provided directly at the diaper or diaper ear 20, e.g., at the top sheet or the inner surface of the diaper ear during the manufacturing process of the diaper. The fastening tab is attached and folded over similarly as described above with respect to Figs. 3a-3c so that the mechanical fastening element 4 is placed onto the covering strip 12, as shown in Fig. 4b. The covering strip 12 may be attached to the diaper ear by an additional layer of adhesive or by welding or spot bonding. When the user's end of the fastening tab is pulled off for use the covering strip 12 remains at the diaper ear and thus the mechanical fastening element 4 is exposed for use as shown in Fig. 4c.

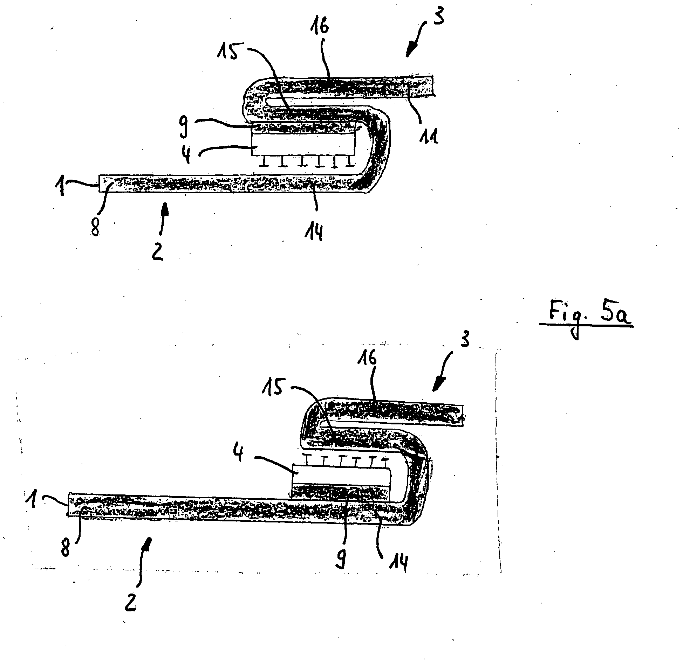

Fig. 5a schematically shows two variations of the embodiment shown in Fig. 2a. Again, the fastening tab comprises a backing 1 with a manufacturer's end 2 and a user's end 3. The backing 1 comprises a substrate layer 8 which is only partially covered with a layer of adhesive 9. At the user's end 3, a mechanical fastening element 4 is attached to the backing 1 by means of adhesive layer 9. Preferably, the backing 1 is provided without adhesive layer 9, but rather the mechanical fastening element 4 is provided with the adhesive layer 9. In this way strip coating (i.e. partial coating) of the backing 1 can be avoided. In this embodiment, the elongated or enlarged fingerlift 11 is provided by the backing 1 itself instead of a separate element protruding therefrom as in the embodiment of Figs. 2a to 2c. The fingerlift 11 provided by the substrate layer 8 of backing 1 is folded either together with the mechanical fastening element 4 (upper version of Fig. 5a) or without the mechanical fastening element 4 (lower version of Fig. 5a) into a Z-shape, wherein the mechanical fastening element 4 is covered by the backing 1 itself.

Thus, in the upper version of Fig. 5a the base portion 14 comprises the backing 1. The middle portion 15 comprises a combination of backing 1 and mechanical fastening element 4. The top portion 16 comprises the backing 1. In the lower version of Fig. 5a, the base portion 14 comprises a combination of the backing 1 and the mechanical fastening element 4, the middle portion 15 comprises the backing 1, and the top portion 16 also comprises the backing 1.

The embodiments shown in Figs. 2a and 5a refer to examples only. Other implementations of a Z-fold are possible as well. For instance, the fingerlift portion 11 of Fig. 2a could be used in the Z folded as shown in Fig. 5a and vice versa. Moreover, the elongated fingerlift portion 11 may be provided in a different manner. Instead of adding a separate portion as in the embodiment of Fig. 2a or forming the fingerlift portion by the substrate layer 8 of backing 1, the backing 1 may be entirely provided with an adhesive layer 9 and some portions thereof may be covered with a blanking film. The fastening tab with such a fingerlift portion could be folded in all ways described above.

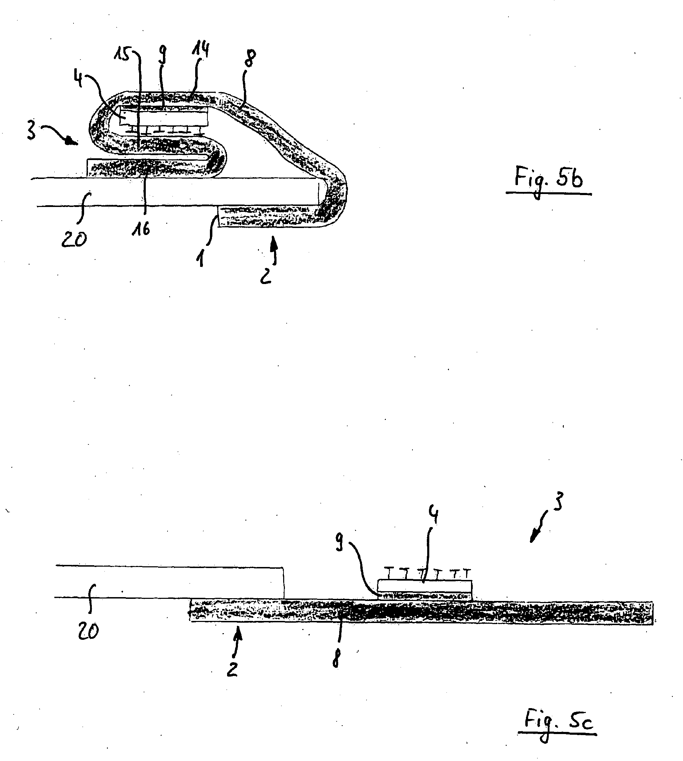

Alike described above, the manufacturer's end 2 of the backing 1 is attached to the diaper ear 20 via an additional adhesive layer (not shown in Fig. 5a) as shown in Fig. 5b. Said adhesive may be either provided on the substrate 8 of the backing 1 or on the diaper ear 20. The user's end 3 of the fastening tab is folded around the diaper ear 20 and attached to it. Upon application of a pulling force onto the user's end 3 of the fastening tab, the Z-shape is released and the covering of the mechanical fastening element 4 is removed so that the mechanical fastening element 4 is exposed for use as shown in Fig. 5c.

The fastening tabs and diapers according to the present invention have several advantages. No or only less fibers can be ripped off from a diaper ear comprising a non-woven material when the mechanical fastening element is exposed for use. At the same time, the present invention allows for secure storage of the fastening tab within the diaper prior to use. This is important during the process of production as well as for packaging purposes (so-called anti-flagging). The fastening tabs and diapers of the present invention are easy to use. The fingerlift portions can be gripped well and torn off with ease. These features are appreciated a lot by parents struggling to keep the fidgeting baby calm with one hand and trying to close the diaper with the other hand. Furthermore, the design of the fastening elements ensures that other benefits are not affected. For instance, the extensibility of stretchable diaper ears is not reduced in diapers according to the present invention.

## Claims

1. A fastening tab for a disposable product, comprising:
a) a backing (1) having a manufacturer's end (2) and an opposite user's end (3),
wherein the fastening tab is adapted to be secured to the disposable product at its manufacturer's end (2), and the user's end (3) comprises a fingerlift (11);
b) a mechanical fastening element (4) attached to the user's end (3) of the backing (1); and
wherein the user's end (3) is folded in a Z-shape such that the mechanical fastening element (4) is at least partially covered by a portion of the user's end (3), and wherein, upon application of a pulling force onto the user's end (3) of the fastening tab, the Z-shaped portion of the user's end (3) is released and the covering of the mechanical fastening element (4) is removed so that the mechanical fastening element (4) is exposed.

2. The fastening tab according to claim 1, wherein the fingerlift (11) protrudes from the backing (1) of the fastening tab.

3. The fastening tab according to claim 1 or 2, wherein the pulling force onto the user's end (3) sufficient for releasing the Z-shaped portion of the user's end (3) exposing the mechanical fastening element (14), is between 0.5 N and 10 N.

4. The fastening tab according to any one of claims 1 to 3, wherein said Z-shaped portion of the user's end (3) comprises a base portion (14), a middle portion (15) and a top portion (16), wherein the base portion (14) is formed by the backing (1) including the mechanical fastening element (4) and the middle and top portions (15,16) are formed by the fingerlift (11) and/or the backing (1).

5. The fastening tab according to any one of claims 1 to 3, wherein said Z-shaped portion of the user's end (3) comprises a base portion (14), a middle portion (15) and a top portion (16), wherein the base portion (14) is formed by the backing (1), the middle portion (15) is formed by the backing (1) including the mechanical fastening element (4), and the top portion (16) is formed by the fingerlift (11) and/or the backing (1).

6. The fastening tab according to any one of claims 1 to 5, further comprising a release tape (5) attached to the backing (1).

7. The fastening tab according to any one of claims 1 to 6, wherein the Z-shaped portion of the user's end (3) is retained by means of spot bonding and/or welding.

8. A fastening tab for a disposable product, comprising a backing (1) having a manufacturer's end (2) and an opposite user's end (3), wherein the fastening tab is adapted to be secured to the disposable product at its manufacturer's end (2); a mechanical fastening element (4) attached to the backing (1) at its user's end (3); and a release tape (5) having a first leg (6) firmly attached to the backing and a second leg (7) folded around a fold line over onto the mechanical fastening element (4), wherein the second leg (7) covers the mechanical fastening element (4).

9. The fastening tab according to claim 8, wherein the second leg (7) of the release tape (5) comprises a non-woven material.

10. The fastening tab according to claim 8 or 9, wherein only a portion of the second leg (7) is adapted to be attached to the disposable product.

11. The fastening tab according to any one of claims 8 to 10, wherein the second leg (7) of the release tape (15) comprises an adhesive (7d) in a first portion adjacent to the fold line for attachment to the disposable product and a second portion (7c) free of adhesive covering the mechanical fastening element.

12. A roll of fastening tape comprising a plurality of fastening tabs according to any one of claims 1 to 11, wherein the fastening tabs are arranged on the roll in a cross-direction so that individual fastening tabs can be cut from the fastening tape.

13. A diaper comprising a back sheet, a top sheet, an absorbent core therebetween, and a fastening tab according to any one of claims 1 to 7, wherein the fastening tab is attached to the diaper at its manufacturer's end (2) and the mechanical fastening element (4) is at least partially covered by the Z-shaped portion of the user's end (3) or the second leg (7) of the release tape (5) so that the mechanical fastening element (4) is prevented from fully engaging the top sheet of the diaper.

14. The diaper according to claim 13, further comprising an elastic diaper ear, wherein the fastening tab is attached to the diaper ear at a distal end portion thereof.

15. The diaper according to claim 13 or 14, wherein the user's end (3) of the fastening tab is releasably attached to the diaper.

16. A diaper comprising a back sheet, a top sheet, an absorbent core therebetween, and a fastening tab; said fastening tab comprising a backing (1) having a manufacturer's end (2) and an opposite user's end (3), wherein the fastening tab is secured to the diaper at its manufacturer's end (2); a mechanical fastening element (4) attached to the backing (1) at its user's end (3); and a covering strip (12) for releasably covering the mechanical fastening element (4), said covering strip (12) being attached to the top sheet of the diaper; wherein the attachment between covering strip (12) and the top sheet is substantially stronger than the attachment between covering strip (12) and mechanical fastening element (4).

17. The diaper according to claim 16, wherein the covering strip (12) comprises a non-woven material.

18. The diaper according to claim 16 or 17, wherein only a portion of the covering strip (12) is attached to the top sheet of the diaper.

19. The diaper according to any one of claims 16 to 18, further comprising an elastic diaper ear, wherein the fastening tab is secured to the diaper ear and the covering strip (12) is attached to the inner surface of the diaper ear.

20. A method of manufacturing a diaper, comprising the steps of:
a) providing a diaper comprising a back sheet, a top sheet and an absorbent core therebetween;
b) providing a fastening tab according to any one of claims 1 to 11;
c) attaching the fastening tab with its manufacturer's end (2) to the diaper; and
d) folding the user's end (3) of the fastening tab around an edge of the diaper.

21. The method of claim 20, further comprising the step of releasably attaching the user's end of the fastening tab to the top sheet of the diaper.

22. A method of manufacturing a diaper, comprising the steps of:
a) providing a diaper comprising a back sheet, a top sheet and an absorbent core therebetween;
b) providing a fastening tab comprising a backing having a manufacturer's end (2) and an opposite user's end (3), a mechanical fastening element (4) attached to the backing (1) at its user's end (3), and a covering strip (12) for covering and optionally releasably attaching the mechanical fastening element (4);
c) securing the manufacturer's end (2) of the fastening tab to the diaper;
d) folding the user's end (3) of the fastening tab around an edge of the diaper; and
e) attaching said covering strip (12) to the top sheet of the diaper;
wherein the attachment between covering strip (12) and the top sheet is substantially stronger than the attachment between the covering strip (12) and the mechanical fastening element (4).

23. A method according to claim 22, wherein the sequence of steps d) and e) is exchanged.

24. A method according to claim 22 or 23, wherein the diaper further comprises an elastic diaper ear, wherein in step c) the fastening tab is secured to the diaper ear and the covering strip (12) is adhered to the inner surface of the diaper ear.
